# EUROPÄISCHE PATENTANMELDUNG

(11) **EP 1 262 472 A1**
(43) Veröffentlichungstag der Anmeldung: **04.12.2002**
(21) Anmeldenummer: 02008781.3
(22) Anmeldetag: 19.04.2002
(51) Int. Cl.: C07C 45/82, C07C 47/544

(54) **Verbessertes Verfahren zur Reinigung und Formulierung von o-Phthaldialdehyd**

(30) Priorität: 15.05.2001 AT 7682001
(71) Anmelder: DSM Fine Chemicals Austria Nfg GmbH & Co KG, 4021 Linz (AT)
(72) Erfinder: Giselbrecht, Karlheinz, 4061 Pasching (AT); Raml, Walter, 4202 Hellmonsödt (AT); Hermanseder, Rudolf, 4624 Pennewang (AT)
(74) Vertreter: Klostermann, Ingrid

(57) **Zusammenfassung**

Verbessertes Verfahren zur Reinigung und Formulierung von o-Phthalaldehyd, bei welchem roher o-Phthalaldehyd, erhalten durch Spaltung eines Acetals von o-Phthalaldehyd mittels saurer Hydrolyse, gegebenenfalls durch Zusatz einer wässrigen, basischen Lösung auf einen pH-Wert von 1 bis 8 gestellt wird, worauf nach erfolgter Phasentrennung von der den Aldehyd enthaltenden Phase unter sehr kurzer thermischer Belastung von unter 1 Minute bei einem Druck zwischen 100 mbar und Normaldruck und einer Temperatur von Raumtemperatur bis 180°C Wasser an einem Dünnschichtverdampfer abdestilliert, anschließend aus dem verbleibenden Destillationssumpf o-Phthalaldehyd unter sehr kurzer thermischer Belastung von unter 1 Minute über Kopf bei einem Druck von 0,5 bis 50 mbar und einer Manteltemperatur von 80 bis 180°C über einen Dünnschichtverdampfer abgezogen wird und die so erhaltene o-Phthalaldehyd-Schmelze bei Normaldruck und einer Temperatur von 60 bis 80°C pelletiert wird, wodurch hellgelber o-Phthalaldehyd in hoher Reinheit und lagerstabiler Form erhalten wird.

## Beschreibung

Phthalaldehyde, wie o-Phthaldialdehyd (OPA), finden in vielen Gebieten beispielsweise als Zwischenprodukte für die Herstellung von Farbstoffen, optischen Aufhellern oder speziellen Polymeren, in der Biozid- oder Fotoindustrie, sowie für die Synthese von Pharmachemikalien Anwendung. Aus diesem Grund sind bereits mehrere Herstellungsvarianten beschrieben. So kann der o-Phthaldialdehyd (OPA) beispielsweise gemäß EP-B-0 147 593 durch Ozonolyse von Napthalin in Methanol und katalytischer Reuktion der dabei entstehenden Peroxide mit anschließender Extraktion bzw. Kristallisation gewonnen werden. Der Nachteil bei diesem Verfahren ist, dass der sich als Nebenprodukt bildende Ester nur schwer und unzureichend vom OPA abgetrennt werden kann.

Weiters stellt OPA eine reaktive Verbindung dar, die thermisch und oxidativ nicht stabil ist und die bei längerer Lagerung zum Verblocken, wodurch langwierige Löseprozesse erforderlich werden, die eventuell zu einer Verfärbung des OPA führen können.

Um den Aldehyd vor ungewollten Umsetzungen zu schützen, wurde in EP-A1-0522 312 die Möglichkeit beschrieben, o-Phthaldialdehydtetraalkylacetale, hergestellt durch elektrochemische Oxidation, als Depotverbindungen einzusetzen.

Aus EP-B-0 839 789 ist weiters bekannt OPA durch säurekatalysierte Acetalbildung mit anschließender Destillation in eine geeignete Depotverbindung, wie etwa in ein Dialkoxyphtalan oder Tetraalkylacetal, zu überführen, woraus im Bedarfsfall nach vollständiger Acetalspaltung durch saure Hydrolyse rohes OPA mit Reinheiten von über 99,5% erhalten wird.

Da rohes OPA, beispielsweise hergestellt nach EP-B-0 839 789, jedoch eine rot orange Farbe aufweist, muss anschließend, nach Entfärben mit beispielsweise Aktivkohle oder Tonsil, noch umkristallisiert werden.

Das feine OPA-Pulver mit einem Schmelzpunkt von 57°C, das durch Kristallisation erhalten wird, tendiert ebenfalls zum Verblocken. Weiters weisen die verschiedenen Chargen keine gleichbleibende Farbe und Qualität auf.

Aufgabe der vorliegenden Erfindung war es daher ein verbessertes Verfahren zur Isolierung und Formulierung von OPA zu finden, wodurch rohes OPA auf einfache und sichere Weise und praktisch quantitativ gereinigt und in eine lagerfähige Form gebracht werden kann, die auch noch nach Jahren nicht verblockt.

Unerwarteterweise konnte diese Aufgabe durch eine Destillation des aus der Acetalspaltung erhaltenen rohen, thermisch instabilen o-Phthalaldehyds am Dünnschichtverdampfer mit anschließender Pelletierung gelöst werden.

Gegenstand der Erfindung ist demnach ein verbessertes Verfahren zur Reinigung und Formulierung von o-Phthalaldehyd, das dadurch gekennzeichnet ist, dass roher o-Phthalaldehyd, erhalten durch Spaltung eines Acetals von o-Phthalaldehyd mittels saurer Hydrolyse,
a) gegebenenfalls durch Zusatz einer wässrigen, basischen Lösung auf einen pH-Wert von 1 bis 8 gestellt wird, worauf nach erfolgter Phasentrennung
b) von der den Aldehyd enthaltenden Phase, unter sehr kurzer thermischer Belastung von unter 1 Minute bei einem Druck zwischen 100 mbar und Normaldruck und einer Temperatur von Raumtemperatur bis 180°C, Wasser an einem Dünnschichtverdampfer abdestilliert und
c) anschließend aus dem verbleibenden Destillationssumpf o-Phthalaldehyd unter sehr kurzer thermischer Belastung von unter 1 Minute über Kopf bei einem Druck von 0,5 bis 50 mbar und einer Manteltemperatur von 80 bis 180°C über einen Dünnschichtverdampfer abgezogen wird und
d) die so erhaltene o-Phthalaldehyd-Schmelze bei Normaldruck und einer Temperatur von 60 bis 80°C pelletiert wird,
   wodurch hellgelber o-Phthalaldehyd in hoher Reinheit und lagerstabiler Form erhalten wird.

Bei dem erfindungsgemäßen Verfahren wird roher o-Phthalaldehyd (OPA) gereinigt und in eine lagerstabile Form überführt.

Als Ausgangsmaterial dient roher OPA, der durch Spaltung eines OPA-Acetals erhalten wird. OPA-Acetale, wie etwa Dialkoxyphtalane oder Tetraalkylacetale, können beispielsweise analog EP-A1-0522 312 oder EP-B-0 839 789 hergestellt werden. Die Spaltung der Acetale erfolgt ebenfalls auf üblichen Weg, analog dem Stand der Technik, beispielsweise über saure Hydrolyse bei einem pH-Wert zwischen 0 und 7, bevorzugt zwischen 0 und 3 mittels Mineralsäuren wie HCI, H₂SO₄, H₃PO₄ oder organischer Säuren wie Essigsäure, Ameisensäure und p-Toluolsulfon- oder Methansulfonsäure. Die Reaktionstemperatur liegt bevorzugt zwischen Raumtemperatur und 100°C. Anschließend wird der sich abspaltende Alkohol und gegebenenfalls die Säure unter Vakuum abdestilliert.

Das so erhaltene, zu reinigende roh-OPA weist einen Gehalt von über 99,5% auf.

Erfindungsgemäß erfolgt sodann gegebenenfalls die Zugabe einer wässrigen, basischen Lösung, sodass ein pH-Wert zwischen 1 bis 8, bevorzugt zwischen 2 und 7, eingestellt wird. Als Base eignen sich Carbonate wie etwa Bicarbonate oder verdünnte Laugen, wie etwa NaOH, KOH u.s.w..

Die Zugabe der wässrigen, basischen Lösung erfolgt bei Normaldruck und bei einer Temperatur von 40 bis 95 °C, bevorzugt von 50 bis 70°C.

Anschließend erfolgt eine Phasentrennung, wobei in der unteren, schweren Phase, die einen Wassergehalt von 0,5% bis 3%, bevorzugt 1,0 bis 2% aufweist, der OPA enthalten ist. Die OPA - enthaltende Phase wird durch Destillieren an einem Dünnschichtverdampfer unter sehr kurzer thermischer Belastung durch Abdestillieren von Wasser bzw. von wässrigem OPA entwässert.

Als Dünnschichtverdampfer eignen sich dabei Fallfilmverdampfer, Kurzwegverdampfer u.s.w.. Bevorzugt wird hierbei ein Fallfilmverdampfer eingesetzt.

Der erste Destillationsschritt erfolgt bei einem Druck von 100 mbar bis Normaldruck, bevorzugt bei einem Druck von 100 bis 250 mbar und besonders bevorzugt bei einem Druck von 150 bis 200 mbar.

Die Reaktionstemperatur liegt dabei bei einer Manteltemperatur zwischen Raumtemperatur und 180°C, bevorzugt zwischen 100°C und 180°C.

Die Verweilzeit im Dünnschichtverdampfer ist sehr kurz und liegt zwischen 10 Sekunden und < 1 Minute.

Bei diesem Destillationsschritt wird Wasser abdestilliert, wobei jedoch auch zumeist etwas OPA mitdestilliert wird. Dieser wässrige OPA kann bei einer nächsten Charge wieder beim pH-Wert - Stellen mit wässriger, basischer Lösung dazugefahren werden, sodass ein Ausbeuteverlust an OPA verhindert wird.

Zurück bleibt der Destillationssumpf, der die Hauptmenge an braun gefärbten OPA, mit einem Wassergehalt von nunmehr lediglich 0,01 bis 0,4 %, bevorzugt bis 0,1%, und einem Gehalt von über 99,5%, enthält.

Dieser Destillationssumpf wird sodann wiederum einer Destillation unter sehr kurzer thermischer Belastung am Dünnschichtverdampfer unterzogen, wobei OPA dieses Mal über Kopf abgezogen wird.

Als Dünnschichtverdampfer eignen sich dabei ebenfalls Fallfilmverdampfer, Kurzwegverdampfer u.s.w.. Bevorzugt wird hierbei ein Kurzwegverdampfer eingesetzt. Die Verweilzeit im Dünnschichtverdampfer ist sehr kurz und liegt zwischen 10 Sek. und < 1 Minute.

Der zweite Destillationsschritt erfolgt bei einem Druck von 0,5 bis 50 mbar, bevorzugt bei einem Druck von 3 bis 15 mbar und besonders bevorzugt bei einem Druck von 5 bis 10 mbar.

Die Temperatur liegt dabei zwischen 80°C und 180°C Manteltemperatur.

Das Destillat enthält den gereinigten, nunmehr hellgelben OPA mit einem Gehalt von über 99,5%. Der Sumpf, in dem jedoch auch noch größere Mengen an OPA enthalten sind, kann zur Vermeidung von Ausbeuteverlusten bei einer darauffolgenden Charge zudosiert werden.

Anschließend an die Destillation erfolgt sodann die Pelletierung bei einer Temperatur von 60°C bis 80°C, bevorzugt zwischen 60 und 70°C und Normaldruck.

Während der Pelletabfüllung wird die Temperatur auf unter 30°C abgekühlt.

Durch das erfindungsgemäße Reinigungsverfahren wird OPA in einer lagerstabilen Form erhalten, die auch nach mehreren Monaten nicht verklumpt. Weiters weist erfindungsgemäß gereinigtes OPA eine gleichbleibende Farbe auf, während bei OPA, das durch Kristallisation gereinigt wird, eine unterschiedliche Färbung auftritt. Durch das erfindungsgemäße Reinigungsverfahren werden außerdem Ausbeuteverluste an OPA vermieden.

### Beispiel 1:

OPA roh aus der Acetalspaltung gemäß EP-B-0 839 789 mit einem GC Gehalt von > 99,5 GC-FI% wurde mit 300 ml Wasser versetzt und bei 50°C bis 60°C mit 10 % iger Bicarbonatlösung von pH 3 auf pH 7 gestellt. Anschließend erfolgte die Trennung der Phasen sehr gut und sehr schnell. Die untere 670 g schwere OPA roh Phase hatte einen Wassergehalt von 1,5 %. Am Dünnschichtverdampfer (DV) (0,12 m² Oberfläche) wurde diese Phase unter sehr kurzer thermischer Belastung (< 1 Minute) durch Abdestillieren von wässrigem OPA, der bei der nächsten Charge wieder beim pH-Stellen dazu gefahren wurde, entwässert (DV Parameter: 180 mbar, 7 bar Dampf, 50 l/h N₂ Gegenstrom und 400 ml/h OPA roh Belastung). Im Sumpf sammelten sich 617g braun gefärbter OPA roh mit einem Wassergehalt von 0,09 % und einem Gehalt von < 99,5%.

Dieser OPA roh DV-Sumpf wurde wiederum über den DV gefahren, dieses Mal jedoch über Kopf (DV Parameter: 9 mbar, 7 bar Dampf, 50 l/h N₂ Gegenstrom und 617 g/h OPA roh Belastung). Im Destillat sammelten sich 580 g hell gelber OPA (85% Ausbeute) mit einem GC Gehalt von > 99,5 %. Der Sumpf (37g) enthielt noch mind. 85% OPA, der bei der nächsten DV-Charge dazu gefahren wurde.

Um der OPA Schmelze eine Form zu geben und ein Verpacken des OPA zu verhindern wurde eine Pelletierung bei Normaldruck und 65°C durchgeführt; die Pelletabfüllung erfolgte bei 25°C.

## Patentansprüche

1. Verbessertes Verfahren zur Reinigung und Formulierung von o-Phthalaldehyd, **dadurch gekennzeichnet, dass** roher o-Phthalaldehyd, erhalten durch Spaltung eines Acetals von o-Phthalaldehyd mittels saurer Hydrolyse,
a) gegebenenfalls durch Zusatz einer wässrigen, basischen Lösung auf einen pH-Wert von 1 bis 8 gestellt wird, worauf nach erfolgter Phasentrennung
b) von der den Aldehyd enthaltenden Phase unter sehr kurzer thermischer Belastung von unter 1 Minute bei einem Druck zwischen 100 mbar und Normaldruck und einer Temperatur von Raumtemperatur bis 180°C Wasser an einem Dünnschichtverdampfer abdestilliert und
c) anschließend aus dem verbleibenden Destillationssumpf o-Phthalaldehyd unter sehr kurzer thermischer Belastung von unter 1 Minute über Kopf bei einem Druck von 0,5 bis 50 mbar und einer Manteltemperatur von 80 bis 180°C über einen Dünnschichtverdampfer abgezogen wird und
d) die so erhaltene o-Phthalaldehyd-Schmelze bei Normaldruck und einer Temperatur von 60 bis 80°C pelletiert wird,
wodurch hellgelber o-Phthalaldehyd in hoher Reinheit und lagerstabiler Form erhalten wird.

2. Verfahren nach Anspruch 1, **dadurch gekennzeichnet, dass** in Schritt a) ein pH-Wert zwischen 2 und 7 durch Zugabe einer wässrigen Carbonat-, NaOH- oder KOH-Lösung eingestellt wird.

3. Verfahren nach Anspruch 1, **dadurch gekennzeichnet, dass** die Zugabe der wässrigen, basischen Lösung bei einer Temperatur zwischen 40°C und 95°C erfolgt.

4. Verfahren nach Anspruch 1, **dadurch gekennzeichnet, dass** in Schritt b) die Destillation bei einem Druck zwischen 100 bis 250 mbar und einer Manteltemperatur zwischen 100°C und 180°C erfolgt.

5. Verfahren nach Anspruch 1, **dadurch gekennzeichnet, dass** bei der Destillation in Schritt b) mit dem Wasser gegebenenfalls mitdestillierter o-Phthalaldehyd gegebenenfalls bei einer nächsten Charge in Schritt a) als wässrige o-Phthalaldehyd-Lösung zudosiert wird.

6. Verfahren nach Anspruch 1, **dadurch gekennzeichnet, dass** aus dem durch Schritt b) erhaltenen Destillationssumpf in Schritt c) o-Phthalaldehyd bei 3 bis 15 mbar über Kopf abdestilliert wird.

7. Verfahren nach Anspruch 1, **dadurch gekennzeichnet, dass** der verbleibende Destillationssumpf aus Schritt c), der gegebenenfalls noch o-Phthalaldehyd enthält, gegebenenfalls bei einer nächsten Charge in Schritt c) zudosiert wird.

8. Verfahren nach Anspruch 1, **dadurch gekennzeichnet, dass** in Schritt d) die Pastellierung bei einer Temperatur zwischen 60°C und 70°C erfolgt, worauf während der Pastillenabfüllung die Temperatur auf unter 30°C abgekühlt wird.
